(19) Europäisches Patentamt / European Patent Office / Office européen des brevets

(11) **EP 4 498 073 A1**

(12) **EUROPEAN PATENT APPLICATION**

(43) Date of publication:
**29.01.2025 Bulletin 2025/05**

(21) Application number: **23188416.4**

(22) Date of filing: **28.07.2023**

(51) International Patent Classification (IPC):
**G01N 22/00** $^{(2006.01)}$    **G01N 33/00** $^{(2006.01)}$
**G01N 33/02** $^{(2006.01)}$

(52) Cooperative Patent Classification (CPC):
**G01N 33/0098; G01N 22/00; G01N 33/025**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Stichting IMEC Nederland
6708 WH Wageningen (NL)**

(72) Inventors:
• **Rahul, Yadav**
  **6708 Wageningen (NL)**
• **Peter, Offermans**
  **5301 Zaltbommel (NL)**
• **Rached, Moalla**
  **6701 Wageningen (NL)**

(74) Representative: **Körfer, Thomas
Mitscherlich PartmbB
Patent- und Rechtsanwälte
Karlstraße 7
80333 München (DE)**

(54) **DEVICE AND METHOD FOR SENSING AN ELECTRICAL PROPERTY OF A TEST SAMPLE**

(57)    A sensing device (100) is provided for sensing an electrical property of a test sample. The sensing device comprises a substrate (101), and a closed loop resonator (106) and an open loop resonator (108) arranged on the substrate (101), and are arranged in a cascade configuration, the open loop resonator (108) comprises a first loop end (109) and a second loop end (110) arranged at an edge of the substrate (101) and are configured to be in contact with the test sample. In this regard, the closed loop resonator (106) is configured to couple in an input excitation and to transfer the input excitation towards the open loop resonator (108). Furthermore, the open loop resonator (108) is configured to generate localized electric fields between the first loop end (109) and the second loop end (110) propagating through the test sample.

Fig. 1

**Description**

The invention relates to the measurement and/or sensing of electrical properties, especially of relative changes in concentration of assimilates and electrical conductivity, especially in plant tissues.

Generally, in the current high-tech horticulture, greenhouse climate control parameters may be steered towards optimal crop production based on the continuous measurement of actual greenhouse climate conditions (e.g., temperature, humidity, light) and weather forecast data. Although there are some plant parameters that can be assessed by commercially available sensors (e.g., leaf thickness with Hall effect sensor, stem diameter using dendrometer, heat impulse based sap flow sensors, weighing gutters, and thermal cameras), actual plant status may mainly be taken into account by expert observation and laboratory analysis of sampled tissue.

So far, the above discussed measurements may be done manually and/or be limited to the destructive sampling of fully expanded leaves, followed by time-consuming (ion) chromatography analysis in the lab. However, there is an increasing demand towards the autonomous control of greenhouses, which may require automated monitoring of plant status. A continuous measurement of mineral and sugar content in plant tissue is of high interest, which may provide insights in mineral nutrition, stress levels, photosynthetic efficiency, plant balance and root health.

Radio frequency (RF) and microwave resonator based-sensors can potentially offer non-destructive and transient monitoring of mineral and sugar content in the plant tissue. For example, the document US 2016/0091544 A1 discloses complex permittivity sensing using high-Q microwave resonators for lossy or non-lossy samples.

Accordingly, an object of the invention is to provide a sensing device and a method for sensing an electrical property of a test sample in a non-destructive manner to address the above-mentioned limitations.

The object is solved by the features of the first independent claim for the sensing device and by the features of the second independent claim for the method. The dependent claims contain further developments.

According to a first aspect of the invention, a sensing device is provided for sensing an electrical property of a test sample. The sensing device comprises a substrate, and a closed loop resonator and an open loop resonator arranged on the substrate, and are arranged in a cascade configuration, the open loop resonator comprises a first loop end and a second loop end arranged at an edge of the substrate and are configured to be in contact with the test sample.

In this regard, the closed loop resonator is configured to couple in an input excitation and to transfer the input excitation towards the open loop resonator. Furthermore, the open loop resonator is configured to generate localized electric fields between the first loop end and the second loop end propagating through the test sample.

For instance, the first loop end and the second loop end of the open loop resonator may be arranged at the edge of the substrate such that the first loop end and the second loop end may be in direct contact with the test sample. The direct contact can be a physical contact with the test sample. Additionally or alternatively, the direct contact can be a dielectric contact with the test sample, e.g., through one or more dielectric media in-between.

Alternatively, the closed loop resonator and the open loop resonator may be arranged on separate coupled substrates. For instance, the closed loop resonator may be arranged on a first substrate and the open loop resonator may be arranged on a second substrate, where the first substrate and the second substrate may be coupled such that the closed loop resonator may couple in the input excitation and may transfer the input excitation towards the open loop resonator. In this regard, the first loop end and the second loop end of the open loop resonator may be arranged at the edge of the second substrate such that the first loop end and the second loop end may be in contact with the test sample.

Advantageously, the arrangement of the first loop end and the second loop end of the open loop resonator at the edge of the substrate may enable non-destructive sensing from the edge side of the substrate with a smaller probing region. For example, the probing region may have an area of about $\lambda/100 \times \lambda/5$ mm$^2$, where $\lambda$ is the wavelength on the substrate. Furthermore, the arrangement of the first loop end and the second loop end of the open loop resonator at the edge of the substrate may allow non-destructive probing of non-conformal surfaces of the test sample.

Preferably, the substrate comprises a top plane and a ground plane, whereby the closed loop resonator and the open loop resonator are arranged on the top plane. In this regard, the sensing device comprises a first metal line adjacent to the first loop end and a second metal line adjacent to the second loop end, each being configured to form a metallic contact or a ground loop between the top plane and the ground plane at the edge of the substrate.

For example, the first metal line and the second metal line may be arranged at the edge of the substrate such that the first metal line and the second metal line may be in direct contact with the test sample. The direct contact can be a physical contact with the test sample. Additionally or alternatively, the direct contact can be a dielectric contact with the test sample, e.g., through one or more dielectric media in-between.

Advantageously, the first metal line and the second metal line at the edge of the substrate, especially adjacent to the first loop end and the second loop end, respectively, of the open loop resonator may act as adjacent grounded trace loops, which may result into a higher intensity of the generated electric fields.

Preferably, the sensing device comprises an input port operably coupled to the closed loop resonator configured to input the input excitation, and an output port operably coupled to the closed loop resonator configured to output a transmission parameter measurement based on a response of the closed loop resonator and/or the open loop resonator.

In this regard, the closed loop resonator is configured to be coupled to the input port and to the output port via one or more transmission lines to generate a passband resonance or a stopband resonance.

For instance, the closed loop resonator may be coupled to the input port and to the output port via separate transmission lines, e.g., magnetically coupled to two respective sides of the closed loop resonator, thereby resulting in a passband transmission spectrum. Alternatively, the closed loop resonator may be magnetically coupled to the input port and to the output port via a single transmission line connecting the input port and the output port, thereby resulting in a stopband transmission spectrum.

Preferably, the closed loop resonator and the open loop resonator are arranged in the cascade configuration via a stepped impedance line configured to match an impedance between the closed loop resonator and the open loop resonator. Advantageously, a power transfer from the closed loop resonator towards the open loop resonator can be maximized.

Preferably, the substrate comprises or is a dielectric material, preferably with a relative permittivity between 2 to 100. Additionally or alternatively, the substrate comprises or is a dielectric material, preferably with a thermal coefficient of dielectric constant between -5 to +5, more preferably between -3 to +3. Advantageously, the effect of a change in environmental temperature, especially on microwave measurements, can be minimized.

Preferably, the sensing device comprises a housing comprising an opening corresponding to the edge of the substrate, the housing is configured to encompass the substrate, whereby the opening is configured to arrange at least the first loop end and the second loop end to be in contact with the test sample. For example, the housing material can be polylactic acid, acrylic, glass, metal cavity, or any ferromagnetic material.

Preferably, the housing comprises a holding arrangement configured to maintain a position of the opening with respect to the test sample. Advantageously, for example, the contact between the sensing device and the test sample can be further secured.

Preferably, the closed loop resonator and the open loop resonator are microwave resonators, especially planar microwave ring resonators. Furthermore, the first loop end and the second loop end of the open loop resonator may be arranged in relation to each other such that the open loop resonator may generate fringing electric fields between the first loop end and the second loop end.

Preferably, the test sample is a plant-based sample, especially a plant stem. Moreover, the electrical property of the test sample is permittivity and/or electrical conductivity of the test sample.

Advantageously, an interaction of electric fields can be facilitated with the plant tissue content to assess changes in permittivity and conductivity, especially corresponding to the changes in sugar content from photosynthesis or respiration and ionic conductivity from mineral uptake, respectively.

According to a second aspect of the invention, a method is provided for sensing an electrical property of a test sample. The method comprises the steps of arranging a closed loop resonator and an open loop resonator on a substrate in a cascade configuration, arranging a first loop end and a second loop end of the open loop resonator at an edge of the substrate to be in contact with the test sample, transferring an input excitation from the closed loop resonator towards the open loop resonator, and generating localized electric fields between the first loop end and the second loop end propagating through the test sample.

Preferably, the method further comprises the steps of arranging the closed loop resonator and the open loop resonator on a top plane of the substrate, and providing a first metal line adjacent to the first loop end and a second metal line adjacent to the second loop end to form a respective metallic contact or a ground loop between the top plane and a ground plane at the edge of the substrate.

Preferably, the method further comprises the steps of inputting the input excitation via an input port operably coupled to the closed loop resonator, and outputting a transmission parameter measurement via an output port operably coupled to the closed loop resonator based on a response of the closed loop resonator and/or the open loop resonator.

It is to be noted that the method according to the second aspect corresponds to the sensing device according to the first aspect and its implementation forms. Accordingly, the method of the second aspect may have corresponding implementation forms. Further, the method of the second aspect achieves the same advantages and effects as the sensing device of the first aspect and its respective implementation forms.

Exemplary embodiments of the invention are now further explained with respect to the drawings by way of example only, and not for limitation. In the drawings:

Fig. 1    shows an exemplary embodiment of the sensing device according to the first aspect of the invention;

Fig. 2    shows a detailed view of the edge of the substrate;

Fig. 3A    shows a first exemplary field distribution through the test sample;

Fig. 3B    shows a second exemplary field distribution through the test sample;

Fig. 4    shows an exemplary housing arrangement for the sensing device;

Fig. 5    shows an exemplary transmission spectrum of the sensing device; and

Fig. 6    shows an exemplary embodiment of the method according to the second aspect of the invention.

Reference will now be made in detail to the embodiments of the present invention, examples of which are illustrated in the accompanying drawings. However, the following embodiments of the present invention may be variously modified and the range of the present invention is not limited by the following embodiments.

Generally, for a planar microwave resonator with $\vec{E}$-field sensing region, a shift in its resonance frequency may depend on the change in permittivity of the test medium, for example by a change in sugar concentration within the plant tissue. The presence of a conductive or lossy medium in the sensing region, such as plant tissue with high mineral (ionic) content, can result in an attenuation of signal magnitude at the resonant frequency. Changes in plant status that may be manifested by changes in sugar or mineral content can thus be monitored by measuring both attributes of the microwave resonance spectrum.

For instance, the microwave resonator structures may be designed using metals and dielectric materials to exhibit electric, magnetic, or guided electromagnetic fields that may be associated with a resonance condition $f_0 = 1/\sqrt{LC}$, where $f_0$ is the resonant frequency, L and C denotes total inductance and capacitance respectively. The resonance condition may also be a function of substrate dielectric properties (i.e., relative permittivity) and the physical dimension of the metallization.

Accordingly, planar microwave resonator designs may be adopted for dielectric sensing and characterization of chemical, biological samples, organic and inorganic compounds, and other metrological applications. In principle, it may work by interacting desired field vectors with the test material in transmission or reflection mode. The perturbation of the field vectors by interaction with test material medium in the reactive or near-field zone may effectively alter the inductance (L) and/or capacitance (C) of the LC-circuit attributed to the microwave structure.

As a result, it may change the effective material medium response (i.e., effective permittivity) of the resonator, resulting in a measurable shift or attenuation in the transmission or reflection spectra. The sensing mechanism can also be understood from the following equation, which can be derived using EM-field perturbation theory for a cavity model and expresses the change in the resonance frequency as:

$$\frac{\Delta\omega_r}{\omega_r} = \frac{\iiint_{vs}(\varepsilon_s - 1)\overrightarrow{E_s^*}.\overrightarrow{E_\iota}dv}{2\iiint_{vc}|\overrightarrow{E_s}|^2dv},$$

where, $\Delta\omega_r$ denotes changes in the resonance of the cavity with sample loading, $\omega_r$ is the fundamental resonance of the cavity without sample loading, $\varepsilon_s$ indicates the complex permittivity of the test sample, $\overrightarrow{E_s^*}$ denotes the electric field subjected to the test sample, $\overrightarrow{E_\iota}$ is the total electric field present in the cavity and $vs$, $vc$ are the volume of sample and the entire cavity respectively.

The change in resonance $\Delta\omega_r$ by the introduction of the sample can be calculated when the $\vec{E}$-field inside the cavity and the sample is known, e.g., from numerical calculation or full-wave simulation. Temperature stability, higher signal to noise ratio, compact size and sensitivity are some of the parameters that can be achieved through distinctive design and optimization.

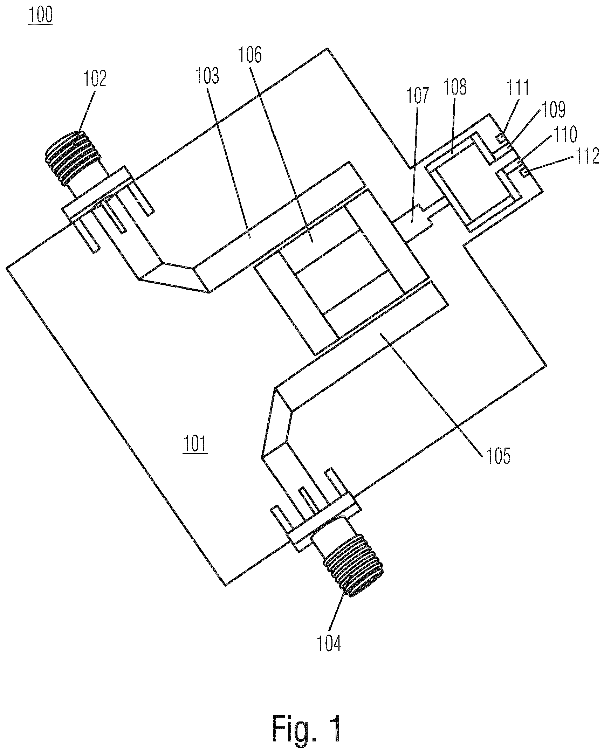

In Fig. 1, an exemplary embodiment of the sensing device 100 according to the first aspect of the invention is illustrated. The sensing device 100 may comprise a substrate 101, an input port 102, and an output port 104, where a input signal line 103 and an output signal line 105, for example, 50 ohm microstrip lines, may be formed on the substrate 101 connected to the input port 102 and the output port 104, respectively. The input port 102 and the output port 104 may comprise or be RF connectors, e.g., SMA connectors.

The sensing device 100 may further comprise a closed loop resonator 106 formed on the substrate 101, especially between the input signal line 103 and the output signal line 105. For instance, the input port 102 may input a microwave signal excitation, whereby the closed loop resonator 106 may use the input signal line 103, e.g., a line length of λ/2 of the input signal line 103, to magnetically couple the energy into the closed loop resonator 106.

Accordingly, the closed loop resonator 106 may generate the transmission spectrum of the microwave signal, and may provide the transmission spectrum at the output port 104 via the magnetically coupled output signal line 105, e.g., a line length of λ/2 of the output signal line 105.

Therefore, in this example, one side of the closed loop resonator 106 may be mutually coupled to the input signal line 103 and one side of the closed loop resonator 106 may be mutually coupled to the output signal line 105.

The sensing device 100 may further comprise an open loop resonator 108 formed on the substrate 101, and may be connected to the closed loop resonator 106 in a cascade configuration via a stepped impedance line or trace 107. For example, the thickness of the microstrip configurations of the closed loop resonator 106 and the open loop resonator 108 may be matched by the stepped impedance line 107, especially to perform impedance matching between the closed loop resonator 106 and the open loop resonator 108.

For instance, the open loop resonator 108 may comprise a first loop end 109 and a second loop end 110, i.e., the loop ends of the open loop resonator 108, which may be extended and/or extruded towards an edge of the substrate 101.

The sensing device 100 may further comprise a first ground loop 111 and a second ground loop 112 adjacent to the first loop end 109 and the second loop end 110, respectively, at the edge of the substrate 101.

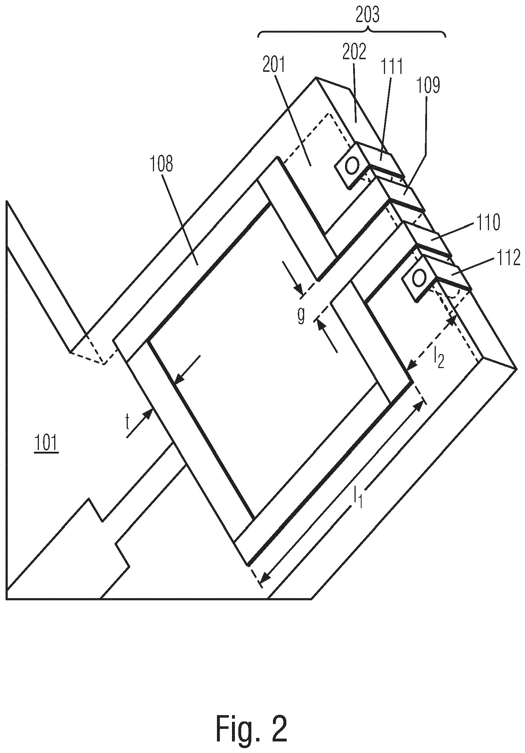

In Fig. 2, a detailed view of the edge of the substrate 101 is illustrated. For example, the substrate 101 may comprise a top plane or a signal plane 201 and a ground plane 202, whereby the input signal line 103, the output signal line 105, the closed loop resonator 106, the stepped impedance line 107, and the open loop resonator 108 may be arranged on the signal plane 201 of the substrate 101.

For example, the open loop resonator 108 may comprise microstrip lines with a thickness t and a length $l_1$, and a gap g between the first loop end 109 and the second loop end 110. Furthermore, the first loop end 109 and the second loop end 110 may be extended by a length $l_2$ towards the edge 203, and be extruded to the edge 203, e.g., trace extrusions to the PCB edge.

For instance, the first ground loop 111 may be arranged at the edge 203 adjacent to the extruded first loop end 109, e.g., via grounded metallization to form a high impedance (~100 ohm) trace extruded via the edge 203 connecting the signal plane 201 and the ground plane 202. Similarly, the second ground loop 112 may be arranged at the edge 203 adjacent to the extruded second loop end 110, e.g., via grounded metallization to form a high impedance (~100 ohm) trace extruded via the edge 203 connecting the signal plane 201 and the ground plane 202.

The sensing device 100 may facilitate a direct contact with a test sample at the edge 203 of the substrate 101, especially to be in contact with the test sample via the extruded first loop end 109 and the second loop end 110, and further via the first ground loop 111 and the second ground loop 112.

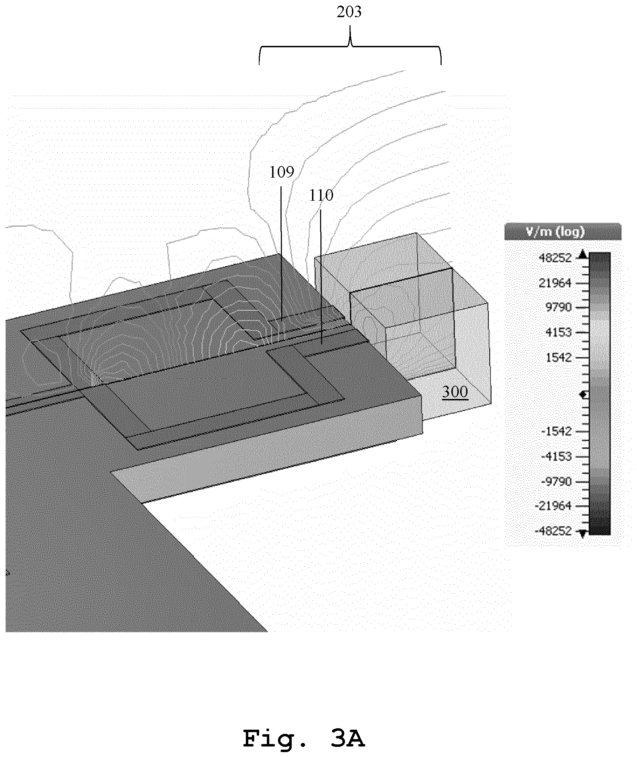

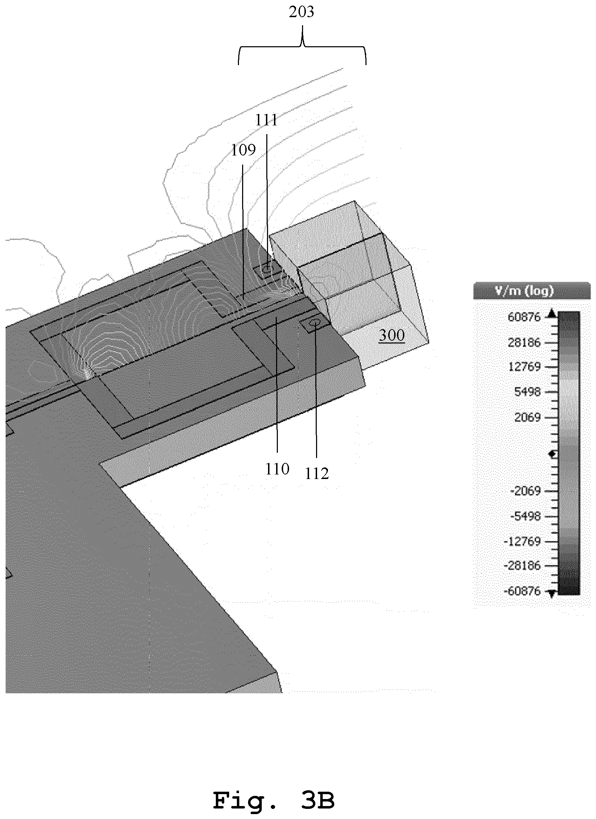

Figs. 3A and 3B show the effect of the ground loops 111, 112 on the electric fields at the edge 203 of the substrate 101. In particular, Fig. 3A shows a first exemplary field distribution through the test sample 300 with no ground loops present at the edge 203, i.e., no adjacent ground loops to the first loop end 109 and the second loop end 110. It can be seen that, the configuration can achieve a maximum electric field intensity of ~48KV/m through the test sample 300.

In Fig. 3B, a second exemplary field distribution through the test sample 300 is illustrated. In this configuration, the first ground loop 111 and the second ground loop 112 are present at the edge 203, and are adjacent to the first loop end 109 and the second loop end 110, respectively. It can be seen that, the configuration can achieve a maximum electric field intensity of ~60KV/m through the test sample 300.

Therefore, the adjacent ground loops 111, 112 improve the directivity and the distribution of the electric fields generated between the loop ends 109, 110 of the open loop resonator 108, especially through the test sample 300.

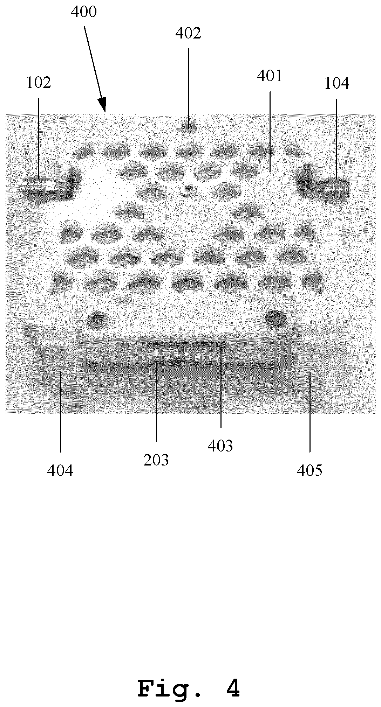

In Fig. 4, an exemplary housing arrangement 400 for the sensing device 100 is illustrated. The housing 400 may comprise a shielding material or surface 401 encompassing the substrate 101. The shielding material 401 may be attached to the substrate 101 in a non-conductive manner via one or more fixing means 402, e.g., comprising glue or screws or adaptive fittings.

The housing 400 may further comprise an opening 403 arranged at the edge 203 of the substrate 101 such that the edge 203, especially comprising the loop ends 109, 110, and the adjacent ground loops 111, 112, may protrude through the opening 403.

The housing 400 may further comprise a holding arrangement 404, 405, for example, comprising one or more clamps or shaped structures, especially arranged near the opening 403 to secure an attachment of the sensing device 100 to the test sample 300.

For example, the shielding material 401 can be polylactic acid, acrylic, glass, metal cavity, or any ferromagnetic material. Furthermore, the substrate 101 may have a relative permittivity of 3 and a thickness of 1.52mm. Moreover, the substrate 101 may have a thermal coefficient of dielectric constant of -3.

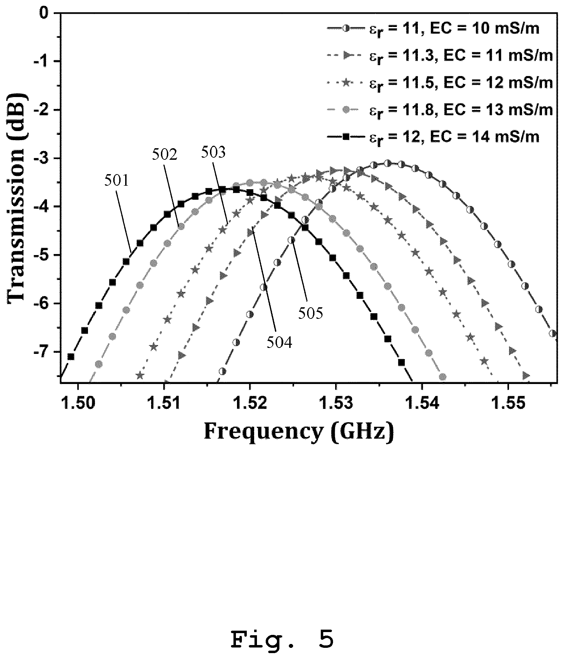

In Fig. 5, an exemplary transmission spectrum of the sensing device 100 is illustrated. In particular, Fig. 5 shows a plot of transmission spectrum for simultaneous changes in permittivity and conductivity in a plant SAP model. The horizontal axis denotes the frequency in GHz and the vertical axis denotes the transmission in dB.

In this regard, the plot 501 corresponds to a relative permittivity of 12 and an electrical conductivity of 14mS/s, the plot 502 corresponds to a relative permittivity of 11.8 and an electrical conductivity of 13mS/s, the plot 503 corresponds to a relative permittivity of 11.5 and an electrical conductivity of 12mS/s, the plot 504 corresponds to a relative permittivity of 11.3 and an electrical conductivity of 11mS/s, and the plot 505 corresponds to a relative permittivity of 11 and an electrical conductivity of 10mS/s.

It can be seen, an increase in the electrical conductivity, i.e., from 10mS/s to 14mS/s, which corresponds to the minerals uptake by the plant tissue, attenuates the transmission magnitude, i.e., the decrease in the magnitude observed from the plot 505 to the plot 501. It can further be seen, a decrease in the relative permittivity, i.e., from 12 to 11, which corresponds to an increase in sugar, shifts the frequency, i.e., the shift in frequency observed from the plot 501 to the plot 505.

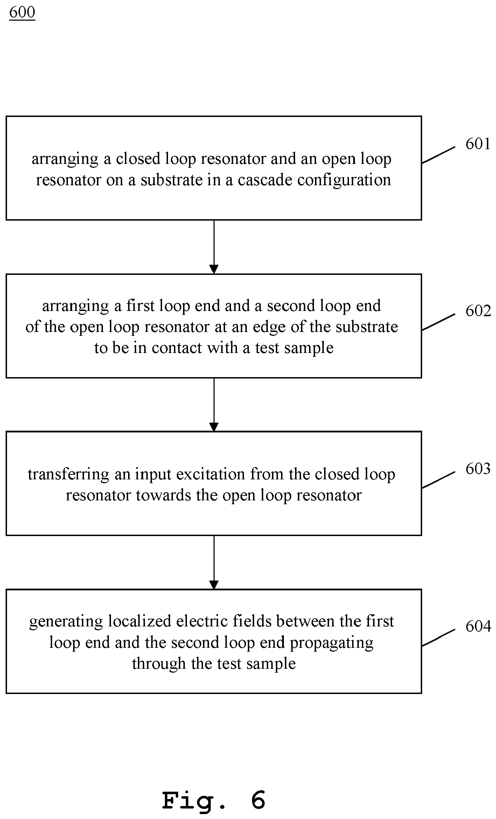

In Fig. 6, an exemplary embodiment of the method 600 according to the second aspect of the invention is illustrated. In a first step 601, a closed loop resonator and an open loop resonator are arranged on a substrate in a cascade configuration. In a second step 602, a first loop end and a second loop end of the open loop resonator are arranged at an edge of the substrate to be in contact with the test sample.

In a third step 603, an input excitation is transferred from the closed loop resonator towards the open loop resonator. In a fourth step 604, localized electric fields are generated between the first loop end and the second loop end propagating through the test sample, especially in response to the transferred input excitation.

Therefore, the invention presents a microwave resonator based sensing scheme using extruded metallization of the ring resonator and additional ground loop trace towards the substrate edge, especially to facilitate localized probing of plant stem tissues of a greenhouse crop in a non-destructive manner.

In particular, the generation of controlled and higher intensity fields at the substrate edge and the use of temperature stable substrate for the resonator design may enable effective monitoring of assimilates and mineral content profile with very minimally being influenced by a change in environmental condition or a physical change in the stem diameter.

This may overcome the limitations in planar microwave resonators, where the region of electric or magnetic field utilized for perturbation with test medium have limited extent of fields into the test medium, whereby achieving a higher sensitivity and a controlled penetration depths into the test medium.

It is important to note that, in the description as well as in the claims, the word "comprising" does not exclude other elements or steps and the indefinite article "a" or "an" does not exclude a plurality. A single element or other unit may fulfill the functions of several entities or items recited in the claims. Furthermore, the word "coupled" implies that the elements may be directly connected together or may be coupled through one or more intervening elements. Moreover, the disclosure with regard to any of the aspects is also relevant with regard to the other aspects of the disclosure.

Although the invention has been illustrated and described with respect to one or more implementations, equivalent alterations and modifications will occur to others skilled in the art upon the reading and understanding of this specification and the annexed drawings. In addition, while a particular feature of the invention may have been disclosed with respect to only one of several implementations, such feature may be combined with one or more other features of the other implementations as may be desired and advantageous for any given or particular application.

**Claims**

1. A sensing device (100) for sensing an electrical property of a test sample, comprising:

   a substrate (101);
   a closed loop resonator (106) and an open loop resonator (108) arranged on the substrate (101) and arranged in a cascade configuration;
   wherein the open loop resonator (108) comprises a first loop end (109) and a second loop end (110), which are arranged at an edge of the substrate (101) and are configured to be in contact with the test sample;
   wherein the closed loop resonator (106) is configured to couple in an input excitation and to transfer the input excitation towards the open loop resonator (108), and
   wherein the open loop resonator (108) is configured to generate localized electric fields between the first loop end (109) and the second loop end (110) propagating through the test sample.

2. The sensing device according to claim 1,

   wherein the substrate (101) comprises a top plane (201) and a ground plane (202), whereby the closed loop resonator (106) and the open loop resonator (108) are arranged on the top plane (201), and
   wherein the sensing device comprises a first metal line (111) adjacent to the first loop end (109) and a second metal line (112) adjacent to the second loop end (110), each being configured to form a metallic contact or a ground loop between the top plane (201) and the ground plane (202) at the edge of the substrate (101).

3. The sensing device according to claim 1 or 2, wherein the sensing device comprises an input port (102) operably coupled to the closed loop resonator (106) configured to input the input excitation, and an output port (104) operably coupled to the closed loop resonator (106) configured to output a transmission parameter measurement based on a response of the closed loop resonator (106) and/or the open loop resonator (108).

4. The sensing device according to claim 3, wherein the closed loop resonator (106) is configured to be coupled to the input port (102) and to the output port (104) via one or more transmission lines (103, 105) to generate a passband resonance or a stopband resonance.

5. The sensing device according to any of claims 1 to 4, wherein the closed loop resonator (106) and the open loop resonator (108) are arranged in the cascade configuration via a stepped impedance line (107) configured to match an impedance between the closed loop resonator (106) and the open loop resonator (108).

6. The sensing device according to any of claims 1 to 5, wherein the substrate (101) comprises or is a dielectric material, preferably with a relative permittivity between 2 to 100.

7. The sensing device according to any of claims 1 to 5, wherein the substrate (101) comprises or is a dielectric material, preferably with a thermal coefficient of dielectric constant between -5 to +5, more preferably between -3 to +3.

8. The sensing device according to any of claims 1 to 7, wherein the sensing device comprises a housing (400) comprising an opening (403) corresponding to the edge of the substrate (101), the housing is configured to encompass the substrate (101), whereby the opening (403) is configured to arrange at least the first loop end (109) and the second loop end (110) to be in contact with the test sample.

9. The sensing device according to claim 8, wherein the housing (400) comprises a holding arrangement (404, 405) configured to maintain a position of the opening (403) with respect to the test sample.

10. The sensing device according to any of claims 1 to 9, wherein the closed loop resonator (106) and the open loop resonator (108) are microwave resonators, especially planar microwave ring resonators.

11. The sensing device according to any of claims 1 to 10, wherein the test sample is a plant based sample, especially a plant stem.

12. The sensing device according to any of claims 1 to 11, wherein the electrical property of the test sample is permittivity and/or electric conductivity of the test sample.

13. A method (600) for sensing an electrical property of a test sample, comprising:

arranging (601) a closed loop resonator and an open loop resonator on a substrate in a cascade configuration,
arranging (602) a first loop end and a second loop end of the open loop resonator at an edge of the substrate to be in contact with the test sample,
transferring (603) an input excitation from the closed loop resonator towards the open loop resonator, and
generating (604) localized electric fields between the first loop end and the second loop end propagating through the test sample.

14. The method according to claim 13, wherein the method further comprising:

arranging the closed loop resonator and the open loop resonator on a top plane of the substrate, and
providing a first metal line adjacent to the first loop end and a second metal line adjacent to the second loop end to form a respective metallic contact or a ground loop between the top plane and a ground plane at the edge of the substrate.

15. The method according to claim 13 or 14, wherein the method further comprising:

inputting the input excitation via an input port operably coupled to the closed loop resonator, and
outputting a transmission parameter measurement via an output port operably coupled to the closed loop resonator based on a response of the closed loop resonator and/or the open loop resonator.

Fig. 1

Fig. 2

Fig. 3A

Fig. 3B

Fig. 4

Fig. 5

600

arranging a closed loop resonator and an open loop resonator on a substrate in a cascade configuration — 601

arranging a first loop end and a second loop end of the open loop resonator at an edge of the substrate to be in contact with a test sample — 602

transferring an input excitation from the closed loop resonator towards the open loop resonator — 603

generating localized electric fields between the first loop end and the second loop end propagating through the test sample — 604

Fig. 6

## EUROPEAN SEARCH REPORT

Application Number

EP 23 18 8416

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KR 2017 0059756 A (UNIV SOGANG RES FOUNDATION [KR]) 31 May 2017 (2017-05-31) * paragraphs [0014], [0023] - [0064]; figures * | 1-15 | INV. G01N22/00 G01N33/00 G01N33/02 |
| A | US 2020/348245 A1 (SHEIKH SHARIF IQBAL MITU [SA] ET AL) 5 November 2020 (2020-11-05) * paragraph [0006] - paragraph [0016]; figures * | 1-15 | |
| A,D | US 2016/091544 A1 (DANESHMAND MOJGAN [CA] ET AL) 31 March 2016 (2016-03-31) * the whole document * | 1-15 | |
| A | CHOON SIK CHO ET AL: "Double Half-Ring Resonators for Bandpass Filter Applications with Suppression of Multiple Harmonics and Size Reduction", MICROWAVE CONFERENCE PROCEEDINGS, 2005. APMC 2005. ASIA-PACIFIC CONFER ENCE PROCEEDINGS SUZHOU, CHINA 04-07 DEC. 2005, PISCATAWAY, NJ, USA,IEEE, vol. 1, 4 December 2005 (2005-12-04), pages 1-4, XP010901801, DOI: 10.1109/APMC.2005.1606303 ISBN: 978-0-7803-9433-9 * the whole document * | 1-15 | TECHNICAL FIELDS SEARCHED (IPC) G01N |
| A | CA 3 016 064 A1 (UNIV MANITOBA [CA]) 29 February 2020 (2020-02-29) * page 1, line 3 - page 2, line 19 * * page 3, line 13 - page 5, line 15 * | 1-15 | |

The present search report has been drawn up for all claims

| Place of search | Date of completion of the search | Examiner |
|---|---|---|
| The Hague | 1 December 2023 | Savage, John |

EPO FORM 1503 03.82 (P04C01)

## ANNEX TO THE EUROPEAN SEARCH REPORT
## ON EUROPEAN PATENT APPLICATION NO.

**EP 23 18 8416**

This annex lists the patent family members relating to the patent documents cited in the above-mentioned European search report.
The members are as contained in the European Patent Office EDP file on
The European Patent Office is in no way liable for these particulars which are merely given for the purpose of information.

**01-12-2023**

| Patent document cited in search report | Publication date | Patent family member(s) | | Publication date |
|---|---|---|---|---|
| KR 20170059756 A | 31-05-2017 | NONE | | |
| US 2020348245 A1 | 05-11-2020 | NONE | | |
| US 2016091544 A1 | 31-03-2016 | CA | 2906268 A1 | 29-03-2016 |
| | | US | 2016091544 A1 | 31-03-2016 |
| | | US | 2019094156 A1 | 28-03-2019 |
| CA 3016064 A1 | 29-02-2020 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20160091544 A1